# EUROPEAN PATENT SPECIFICATION

(11) **EP 1 362 558 B1**
(45) Date of publication and mention of the grant of the patent: **15.03.2006**
(21) Application number: 02009061.9
(22) Date of filing: 23.04.2002
(51) Int. Cl.: A61B 19/08

(54) **Surgical drape**
Chirurgisches Abdecktuch
Champ opératoire

(43) Date of publication of application: 19.11.2003
(73) Proprietor: Rotecno AG, 68555 Stabio (CH)
(72) Inventor: Wiedner, Günter, Dr., 82402 Seeshaupt (DE); Duncan, John A., Fife Scotland KY15 7TN (GB); Patel, Sureshchandra R., Dr., Fife Scotland KY11 9XZ (GB)
(74) Representative: Manitz, Finsterwald & Partner GbR

(56) References cited:
- WO-A-97/35530
- US-A- 5 203 350
- US-A- 5 339 831

## Description

The present invention relates to a surgical drape formed from at least one panel material having a first side disposed in use adjacent a patient and a second side facing away from the patient, a taping strip or zone at said first side of said panel and extending parallel to and adjacent to an edge of said drape disposed in use adjacent to a site of a surgical wound, said taping strip being adhesively connectable in use to a patient, for example by the use of a two sided adhesive tape adhered at one side to the patient and at the other side to said taping strip or zone, wherein the taping strip comprises a single kind of medical fabric, preferably a single layer of a medical fabric, at least over a major portion of its width, having a weave or structure sufficiently dense to prevent the passage of bacteria through it, wherein the material forming the taping strip has a portion folded back on itself at a fold towards the second side of the panel and wherein any stitched seam in the folded back portion does not pass through the panel material directly adjacent to the patient. A surgical drape of this kind is known from US-A-5,203,350.

Surgical drapes are well known in the medical community and serve to prevent contamination of the disinfected operation site by foreign bodies, in particular micro-organisms. Two important potential sources of infection of the patient are the transfer of bacteria originating from not disinfected areas of his own body to the exposed tissue at the operation site, and the airborne transfer of bacteria from other sources to the operation site, such as lint or skin particles originating from the surgeon or other staff in the operating theatre.

In the prior art, a wide variety of draping systems have been employed to improve sterility at the site of an operation. It is helpful to consider the features of these diverse systems to gain an appreciation of the object and subject of the present invention.

There are two main classes of surgical drapes, reusable drapes and disposable drapes. Before considering the action of drapes, it should at first be appreciated that micro-organisms can only be transmitted in a medium, they do not move freely on their own, i.e. they are transferred by fluids, aerosol droplets, lint, dust particles, skin particles or the like.

Historically cotton drapes were and still are used as they are absorbent and soak up liquids. However, they provide no bacterial barrier. Even when replaced by polyester cotton drapes treated with a liquid repellent finish, the properties are limited and are lost after a few uses. Both cotton and polyester cotton drapes are lint producers from new and this production increases with each use. Latter-day fabrics for reusable drapes, such as are obtainable from ROTECNO AG, which similarly present a sterile barrier to prevent infection, have improved properties in a number of respects. For instance the fabric is liquid repellent, thereby still functioning as a sterile barrier when contacted by liquids, is non-particle generating, and provided with a grid of crossed conductive fibres to prevent the buildup of static electricity, which, when present, can attract airborne particles and, on discharge, damage sensitive electrical equipment.

In use, such drapes are arranged around the operation site and held in position with clips which grip through the fabric onto the flesh of the patient. A single drape may be used, in which case there is an aperture in the drape which is appropriately dimensioned to allow access to the operation site. If a plurality of drapes are used, these may have a simple rectangular shape and are laid over the patient and overlap each other to form, for instance, a rectangular access area around the operation site. However, with such draping systems it is difficult to efficiently seal off the operation site from the surrounding non-sterile regions of the patient, as openings remain between the patient's skin and the drape through which micro-organisms can be freely carried. One particularly undesirable mode of patient self-infection, is when fluids spilt during the operation flow under or through the drape to non-sterile regions of the patient, become contaminated and then return to the operation site, for instance by capillary action or under the application of pressure to the drapes.

These fabric drapes do possess the key advantage that they are reusable; the fabric construction being suitable for typical hospital cleansing methods such as laundering followed by steam sterilisation. However, over a period of time, the clipped regions of the fabric become damaged, thereby leading to further degradation of the operational conditions.

To avoid some of the shortcomings of clipping drapes, adhesive tapes can be applied to the edges of the drapes to adhere them to the patient's skin.

After laundering the reusable drapes, double-sided adhesive tape, which has a backing paper, for instance siliconised paper, is attached to the edge of the drape. The reusable drape together with the tape now one-sidely adhered to it, is then sterilised, usually steam sterilised at, for instance, 134°C. Prior to the operation, the backing paper is pulled off the adhesive tape and the drape is adhered to the patient's skin at the site of the operation.

As an alternative method, disposable drapes are also used; they are typically comprised of a non-woven cellulosic material. In some versions, the central part of the disposable drape is adhered to the operation site and such drapes can be provided with an absorbent upper surface which is reinforced with plastic under the absorbent layer to act as a bacterial barrier.

Although the best of these drapes may provide an acceptable bacterial barrier, drapes of this kind are not intended to be cleaned or reused. Given the high throughput achieved in a modem operating theatre, the use of such disposable drapes leads to the creation of large amounts of waste material requiring incineration with the ensuing undesirable costs and environmental consequences.

Therefore, it can be seen that a large number of approaches have been used in prior art draping arrangements and systems, each individual approach offering certain advantages but always associated with certain other disadvantages.

One general problem for all draping systems is that the number and variety of special drape designs required to meet all types of operations results in considerable costs, large storage space utilisation and great administrative effort required to efficiently maintain adequate stocks of these items ready for operations.

Having regard to these various considerations, many hospitals and clinics where operations are carried out prefer to use reusable, generally rectangular drapes which are adhered to the patient's skin at the site of the operation, typically by two-sided adhesive tape. Such drapes mean that only a restricted number of drapes of different sizes have to be stored and can be used for a whole variety of different surgical operations. Since they can be reused, they are also cost-effective, despite the relatively high cost of the two-sided tape.

Conventional reusable drapes adhered by two-sided tape to the patient can, however, give rise to contamination of the wound, which is clearly undesirable. A principal problem with such reusable drapes lies in the fact that they are composite constructions which, due to differential shrinkage of the different materials used in the drape when heated and washed, lead to slight crease formation in the region of the taping strip so that the two-sided adhesive tape that is used is not actually fully bonded to the drape but rather capillary channels exist in the vicinity of the creases which result in a bacteria transfer from the region around the site of the operation into the surgical wound. For example, liquids associated with the surgical wound, which can be body fluids of the patient or flushing fluids used during the operation, can flow through the capillary passages and thus come into contact with the patient at areas which are not completely disinfected. There is then the danger that such liquids, having been contaminated with bacteria, flow back through the capillary regions, for example when pressure is exerted on the drape during the operation by movements of the surgeon or the surgical team or by contact with surgical apparatus, so that the contaminated fluid has a chance of reaching the surgical wound and leading to the highly undesirable infection of the surgical wound. This problem can arise with the known surgical drape of US-A-5,203,350.

In addition, stitched seams are frequently present in existing drapes which permit body fluids or fluids used during the operation to migrate through the fine stitch holes of the seams to the exterior or interior of the drape where they can come into contact with other sources of bacterial infection, such as bacteria arising from the surgeon or his team, which, despite all measures taken to ensure complete sterility, are nevertheless present in practice. Such contaminated liquids can also migrate back to the surgical wound and cause infection to the patient.

Regulations (i.g. EN 13795) will shortly enter into force, which require such surgical drapes to withstand certain, relatively high liquid pressures without permitting the transfer of liquids through the fabrics of the drape.

The principal object underlying the present invention is to provide a surgical drape of the initially named kind which does not suffer from shrinkage in the taping strip and thus avoids this cause of the formation of capillary passages, thus significantly reducing the danger of contamination via this route, which minimises the danger of bacteria transfer through seams in the surgical drape, which meets the requirements for withstanding liquid pressures and which can be reused many times without loosing these advantageous properties. It is a further object of the present invention to provide a draping system which is cost-effective in use.

In order to satisfy these objects there is provided a surgical drape of the initially named kind which is characterised in that the material of the taping strip at the folded back portion lies either directly opposite the material of the taping strip at said second side or in that a double thickness of said panel material resulting from said fold at said edge of said drape lies between said taping strip and said folded back portion thereof.

The present invention reflects the recognition that the problem with the known surgical drape of US-A-5,203,350 is that the layer of weldable foil which is placed between the fabric and the folded back portion thereof at the position of the taping strip and welded to them, with the intention of producing a genuinely fluid tight joint, results in differential shrinkage between the foil and the material of the woven fabric during laundering. This differential shrinkage results in creases so that as described above the double sided tape cannot be fully adhered to the fabric but results in capillary passages which can allow liquids to pass along them and infect the surgical wound.

Since, in the present invention, the taping strip is formed of a single layer of medical fabric that is folded back on itself, so that the material of the taping strip at the folded back portion lies either directly opposite the material of the taping strip at said second side, or so that a double thickness of said panel material resulting from said fold at said edge of said drape lies between said taping strip and said folded back portion thereof, no weldable foil is present and there is no danger of differential shrinkage occurring in the region of the taping strip, so that the formation of creases and thus of capillary passages in this region is extensively avoided. Since the seam in the folded back portion does not pass through the panel material directly adjacent to the patient, the problem of fluid and bacteria transfer through the seams is also largely suppressed, so that this source of contamination is avoided.

The panel material itself can readily be made of a material which satisfies the pressure regulations, and the avoidance of stitched seams in the folded back portion, which pass through the panel material directly adjacent to the patient, prevents this leakage path which would otherwise prevent the pressure regulations being met.

Because the drape does not suffer from shrinkage in repeated use at the critical regions adjacent to the site of the surgical wound, its working life is prolonged, thus improving the cost effectiveness of the system.

Since the drape is connected to the patient by adhesive tape, or by the use of spray-on adhesive applied to the taping strip or zone (so that the taping strip or zone should then be considered simply as an adhesive strip or zone), the use of clips in the region of the edge adjacent the surgical wound can be largely avoided so that damage to the fabric in this region is also not to be expected. Clips may admittedly be used to secure the drape to the patient or to the operating table at positions remote from the site of the wound; however, these positions are not as critical as the region directly adjacent to the surgical wound.

In one advantageous form of the invention, the taping strip is defined by a zone of the panel and the folded back portion is a folded back portion of the panel with the fold defining said edge.

This is a simple, but highly effective way of achieving the objects of the invention.

The folded back portion of the panel is expediently sewed at an edge zone of the panel spaced from said edge of the said taping strip formed by the fold of the panel to a further fabric or fabric structure overlying a layer of said panel adjacent to said patient. Said further medical fabric or fabric structure thus forms a pocket with said layer of panel material adjacent the patient. Because the pocket is open at the side remote from said edge, there is no problem with differential shrinkage arising between the further medical fabric or fabric structure and the panel material adjacent the patient although the further medical fabric or fabric structure may be made of a different material or construction from the panel material adjacent the patient.

The further medical fabric can be an absorbent medical fabric which has a high propensity to absorb and retain fluids escaping from the patient or fluids used during the operation, thus substantially preventing such liquids being returned to the surgical wound and potentially contaminating the wound.

The absorbent medical fabric can lie directly opposite to the second side of the panel, since the panel is in any event made of a fabric which has a high resistance to the passage of liquids through it, even under pressure, so that the danger of fluids contained in the absorbent medical fabric penetrating the panel and reaching relatively non-sterile regions of the patient does not exist.

Should operations be carried out which involve large quantities of body fluids or fluids used for the operation, then the surgeon or the surgical team also has the possibility of inserting further absorbent material into the pocket formed between the panel and the absorbent medical fabric.

Another alternative is for the absorbent medical fabric to lie over a part of the folded back portion of the panel. In this case, two layers of panel material are present between the absorbent medical fabric and the patient, thus further reducing the possibility of fluid transfer through the panel to the patient.

The further medical fabric or fabric structure can also be a laminate of at least first and second layers and comprise a first liquid repellent fabric layer overlying the panel material adjacent the patient and a second layer of an absorbent medical fabric. Two high quality liquid repellent layers are then also present; namely, the liquid repellent fabric layer and panel material, thus again reducing the possibility of fluid transfer to the patient's skin and in the reverse direction leading to contamination of the surgical wound.

The further fabric structure may also be a laminate, such as a silicon laminate, having an absorbent surface.

The laminate can, for example, comprise a panel of tightly woven medical fabric impervious to bacteria, for example ROTECNO 2000, at a side of said laminate overlying said panel adjacent to said patient, a panel of absorbent material overlying said panel of tightly woven fabric and a strip of tightly woven medical fabric adjacent said edge of said taping strip and connected by a seam to said absorbent fabric at a distance from said edge, said distance preferably being greater than 2 cm, but typically less than 6 cm.

In an alternative construction, said taping strip is formed by a strip of material folded around an edge fold of said panel and joined to a folded back portion of said panel at a position spaced from said edge fold, said distance preferably being greater than 2 cm but typically being less than 6 cm. Again, the seam between the extra strip of material forming the taping strip and the drape does not penetrate the panel to the first side thereof, since the seam is only made with the folded back portion of the panel. Thus there is no danger of fluid transfer through this seam to the patient and back again.

Preferred embodiments of the invention are set forth in the subordinate claims and in the further description.

The invention will now be described in further detail with reference to preferred embodiments as illustrated by way of example only in the accompanying drawings.

In the drawings there are shown:
- Fig. 1A: a plan view of a first surgical drape in accordance with the present invention at the side remote from the patient,
- Figs. 1B to 1E: sections through the surgical drape of Fig. 1A at the section planes B-B, C-C, D-D and E-E respectively,
- Fig. 2A: a plan view of a second embodiment of a surgical drape in accordance with the present invention,
- Fig. 2B: a section on the section plane B-B of Fig. 2A, and
- Fig. 2C: a sketch showing the surgical drape of Figs. 2A and 2B draped over the body of the patient adjacent to a surgical wound,
- Fig. 2D: a sketch showing four drapes arranged around the site of a surgical wound,
- Fig. 3A: a plan view of a third embodiment of a surgical drape in accordance with the present invention corresponding to the view of Fig. 1A,
- Fig. 3B: a cross-section through the surgical drape of Fig. 3A on the section plane B-B,
- Fig. 4A: a plan view of a fourth embodiment of a surgical drape in accordance with the present invention corresponding to the view of Fig. 1A,
- Fig. 4B: a cross-section through the surgical drape of Fig. 4A on the section plane B-B,
- Fig. 5A: a plan view of a fifth embodiment of a surgical drape in accordance with the present invention corresponding to the view of Fig. 1A,
- Fig. 5B: a cross-section through the surgical drape of Fig. 5A on the section plane B-B,
- Fig. 6A: a plan view of a sixth embodiment of a surgical drape in accordance with the present invention corresponding to the view of Fig. 1A,
- Fig. 6B: a cross-section through the surgical drape of Fig. 6A on the section plane B-B,
- Fig. 7A: a plan view of a seventh embodiment of a surgical drape in accordance with the present invention corresponding to the view of Fig. 1A and
- Fig. 7B: a cross-section through the surgical drape of Fig. 7A on the section plane B-B,

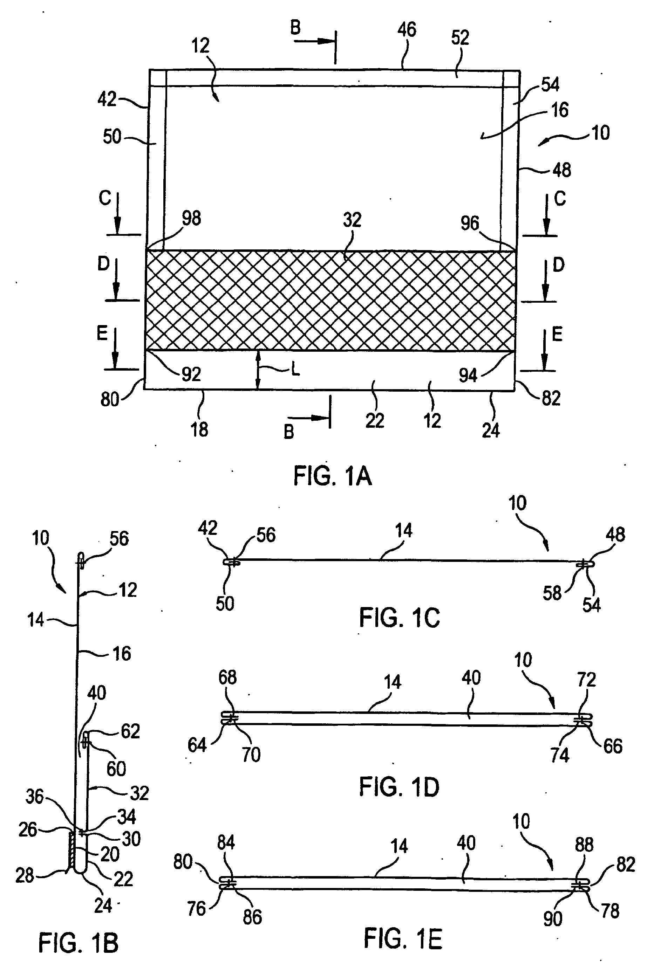

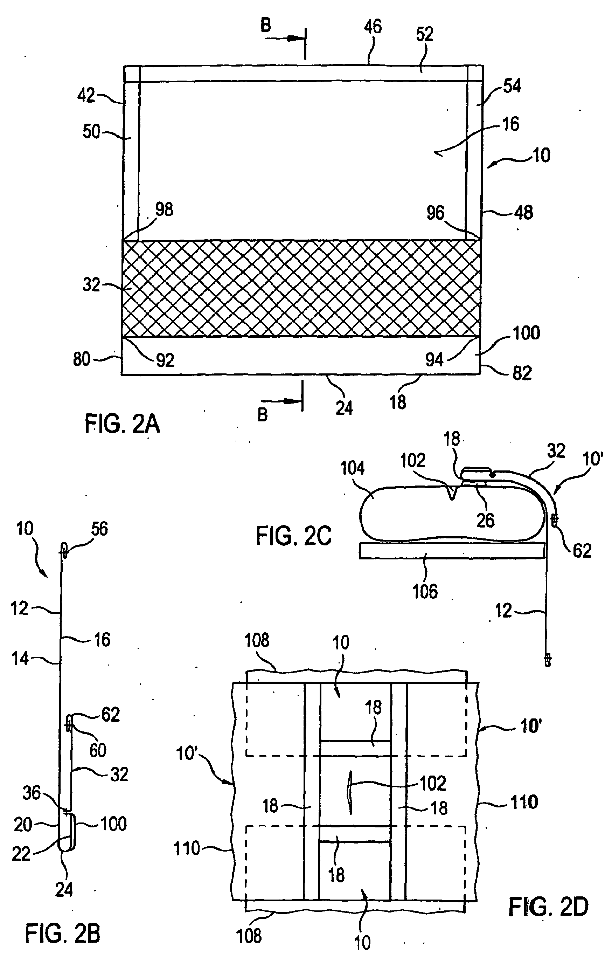

Turning now to Fig. 1A there can be seen a plan view of a surgical drape 10 formed from a panel material 12 having a first side 14 disposed in use adjacent a patient and a second side 16 facing away from the patient. The surgical drape 10 has an edge 18 which is disposed in use adjacent to a site of a surgical wound, as can be seen from the drawings of Figs. 2C and 2D which will be explained in more detail later. The panel material 12 has a taping strip 20. In this embodiment the taping strip, is not a separate physically defined strip but is instead a zone of the panel material 12 disposed adjacent to and extending parallel to and along the edge 18 of the drape disposed in use adjacent to a site of a surgical wound. The panel material forming the taping strip has a portion 22 folded back on itself forming a fold 24, with the fold 24 defining the edge 18.

As can be seen from Fig. 1B a two-sided adhesive tape 26 is adhered to the taping strip 20 at the first side 14 of the panel 12 and has a protective strip 28 which can be peeled off immediately prior to use to free an adhesive surface of the tape for the bonding of the drape to the patient adjacent the site of a surgical wound.

The use of an adhesive tape is not essential, instead adhesive can be applied to the drape at the position of the taping strip or zone and the taping strip or zone adhered directly to the patient by the adhesive.

Attached to the folded back portion 22 of the panel at its edge remote from the fold 24 is a further panel of material 32 with the stitches of the seam between the edge 34 of the further panel 32 and the edge 30 of the folded back portion 22 being indicated by the reference numeral 36. Although the fold 24 is shown as a rounded structure in Fig. 1B, in practice it will be a sharp crease, the illustration of Fig. 1B has simply been opened out somewhat to show the construction of the fabric. It will be noted that the stitches 36, i.e. the stitches of the seam between the edge 34 of the fabric 32 and the edge 30 of the folded back portion 22 of the panel 12 do not penetrate the panel 12 at the part of the panel adjacent the patient, accordingly, there is no danger of contamination being transferred from the patient through the stitching to the site of the wound.

It will also be noted that the taping strip 20 is a strip of a single material so that no problems of differential shrinkage can arise during laundering and/ or sterilisation, so that the danger of creases arising which lead to capillary passages between the fabric and the adhesive strip 26 are avoided. The panel material 12 comprises a liquid repellent, tightly woven fabric which prevents the passage of liquid through it even under relatively high pressure heads, such as 0.5 m of water column. The material used for the panel 12 is, however, preferably permeable to vapour which reduces the danger of perspiration forming on the patient and itself forming a liquid which could potentially be transferred to the site of the surgical wound. The fabric is also made antistatic in known manner. It can, for example, be formed of ROCOT material available from the ROTECNO company under their article number 750.44.0.0.

The material of the medical fabric 32 is preferably ROTECNO-sorbent, i.e. an absorbent medical fabric available from the company ROTECNO under the article number 716.36.0.0, or an absorbent medical fabric as otherwise described in the European patent 710 303. It will be noted that the pocket 40 formed between the panel 12 and the fabric 32 is an open pocket and, if necessary, absorbent material, such as absorbent towels or the like, can be inserted into the pocket to further contain liquids should the absorbent capabilities of the fabric 32 be exceeded.

The three edges 42, 46 and 48 of the drape are provided with relatively simple seams with thoroughgoing stitching as indicated at 56 and 58 since these regions are remote from the portion of the edge 18 adjacent to the site of the surgical wound and there is essentially no danger of bacteria transfer taking place in these regions. The stitching 60 at the edge 62 of the fabric 32 remote from the edge 18 is also executed as thoroughgoing stitching because there is no real danger of contamination arising at this position.

In contrast, the stitching 64 and 66 between the edges of the fabric 32 and the panel 12 is effected at in-turned seams, so that the stitching does not penetrate either fabric towards the outside of the drape. This construction is made physically by first stitching the material as if the (not yet formed) pocket 40 were turned inside out whereupon the two edge portions 68, 70 and 72, 74 of the fabric 32 and the panel 12 can be sewn together. If the so formed pocket is then turned inside out, thus forming the actual pocket 40, then the construction shown in Fig. 1D arises. The edges 68, 70, 72 and 74 are simply cut edges which are prevented from fraying by a zigzag hemming stitch.

Fig. 1E shows a similar section to Fig. 1D, but here between the edges 80 and 82 of the folded back portion 22 and the panel material 12 adjacent to the patient at the ends of the taping strip 20. Again, the stitching 76, 78 does not pass through the panel material 12 adjacent to the patient or through the front portion of the folded back portion 22. The stitching 76, 78 is realised in the same manner as the stitching 64, 66 and again placed between the edges 84, 86, 88 and 90 of the panel material 12 which are located inside the pocket 40 in use. Again, these edges 84, 86, 88 and 90 are cut edges provided with a zigzag hemming stitch to prevent fraying.

The edge seams 50, 52 and 54 in Fig 1A are turned back on themselves as can be seen from Fig. 1C so that fraying at these edges is also not a problem. The stitching 56 at the edge seam 52 of the panel 12 remote from the edge 18 and the stitching 60 provided at the edge 62 of the panel material 12 are stitched right through as through-going stitches at these positions are not critical. The transitions from the absorbent fabric 32 to the panel material 12 at the four corners 92, 94, 96 and 98 can be made in any convenient manner since these four corners are all sufficiently far removed from the site of the surgical wound that they do not constitute a problem with regard to bacteria transfer to the surgical wound. The length L of the folded back portion 22 is not critical, the ideal value would be between 2 and 5 cm, however, it could be made 6 cm or larger if desired.

Turning now to Figs. 2A and 2B there can be seen a second embodiment of a surgical drape in accordance with the present invention which is quite similar to that of Figs. 1A to 1E. The same reference numerals as are used in Figs. 1A to 1E are also used for the same or comparable elements in Figs. 2A to 2B. Thus, the description given to Figs. 1A to 1E applies equally to the construction of Figs. 2A and 2B wherever common reference numerals or letters are used (and also to the diagrams of Figs. 2C and 2D showing the use of the surgical drape during an operation), and it is therefore not necessary to repeat this description again. New reference numerals are used in Figs. 2A to 2D wherever new feature or elements require description. This same convention will also be used for the further embodiments described with reference to the further drawings.

The nature of the seams at the sides 42, 46 and 48 of the surgical drape were discussed in detail in connection with the embodiment of Fig. 1 and that description also basically applies here and to the further embodiments. Such description will not be repeated here because there is essentially no danger of contamination arising from these regions of the surgical drape. This also applies to the constructions described with reference to the further drawings.

There are two differences between the embodiment of Figs. 2A and 2B and that of Figs. 1A to 1E. The first is the material of the medical fabric 32. In this case the medical fabric 32 comprises a laminate with an absorbent surface and a liquid impermeable barrier.

In addition there is also a small width 100 of ROCOT fabric on the folded back portion 22 adjacent to the wound. This acts as a non-wicking section and supplements the non-wicking properties of the ROCOT panel material 12 forming the folded back portion, as in Fig. 1, i.e. the folded back portion 22 and/or the additional strip 100 act to prevent fluids absorbed in the surface of the fabric 32 from passing to the surgical wound by wicking.

Figs. 2C and 2D show how the drapes are arranged in use. The reference numeral 102 shows the surgical wound in a patient 104 lying on an operating theatre table 106. Two drapes 10 are arranged across the patient above and below the surgical wound 102 as shown in Fig. 2D. For space reasons only the portions of the drapes 10 in the vicinity of the edge 18 and the absorbent fabric 32 are shown, the wavy lines 108 simply indicate that the drawing of the drape has been truncated at this point.

Two further drapes 10' extend along the length of the patient's body and overlap the first two drapes 10. Only one of these drapes 10' is shown in Fig. 2C and it can be seen that the drape 10' falls over the sides of the operating table 106 and that the edge 62 of the fabric 32 finishes somewhat above the operating theatre table 106. The lines 110 represent the draped shape of the surgical drapes 10' as they drape over the sides of the patient's body.

It is not essential for four drapes to be used to form a square or rectangular area around the surgical wound. It would, for example, be equally possible to use three drapes defining a generally triangular area. Moreover, it is also possible to use the drapes of the present invention in combination with an incision foil typically provided over the site of the surgical wound.

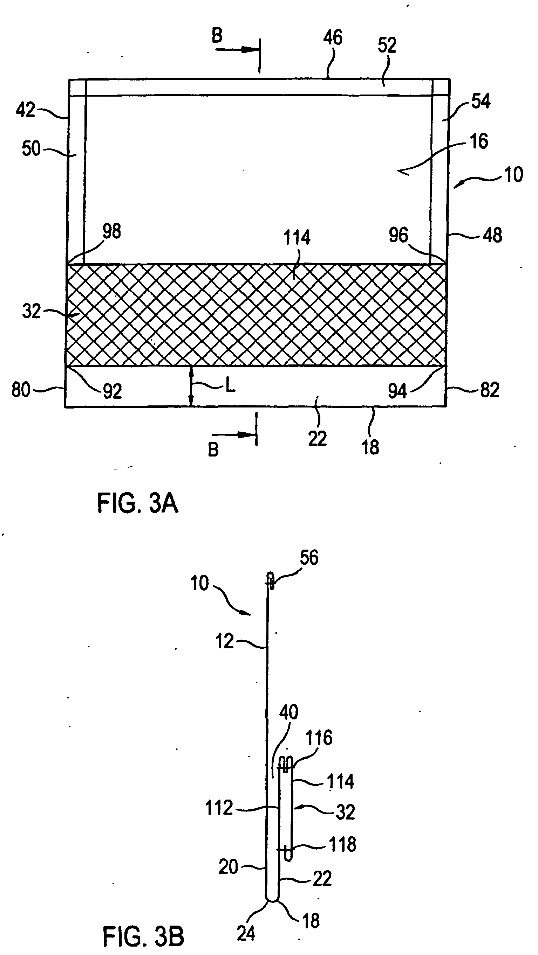

Turning now to Figs. 3A and 3B there can be seen a surgical drape which is again basically similar to the surgical drapes of Figs. 1A to 1E and Figs. 2A to 2B, except that in this case the panel 32 comprises first and second layers 112, 114, namely a first liquid repellent fabric layer 112 overlying the panel material 12 adjacent said patient and a second layer of an absorbent medical fabric at the side of the liquid repellent fabric layer remote from the patient. The way in which the first and second layers 112, 114 of the fabric 32 are stitched to one another can be seen from Fig. 3B and it will be noted that in both cases the stitching 116, 118 passes right through. Again, the turned-in edges are cut edges which are provided with a hemming stitch to prevent fraying. Although it might be thought that the stitching 118 represents a possible contamination path, this is not a problem in practise because the stitching 118 is sufficiently removed from the edge 18. Moreover, the stitching 118 only communicates with the inside of the pocket 40 so that there is no passage from the patient to the stitching 118. In this case the first layer 112 (formed by the panel material 12) is ROCOT article number 750.44.0.0 as described in connection with Fig. 1 and the absorbent layer 114 is ROTECNO sorbent, article no. 716.36.0.0.

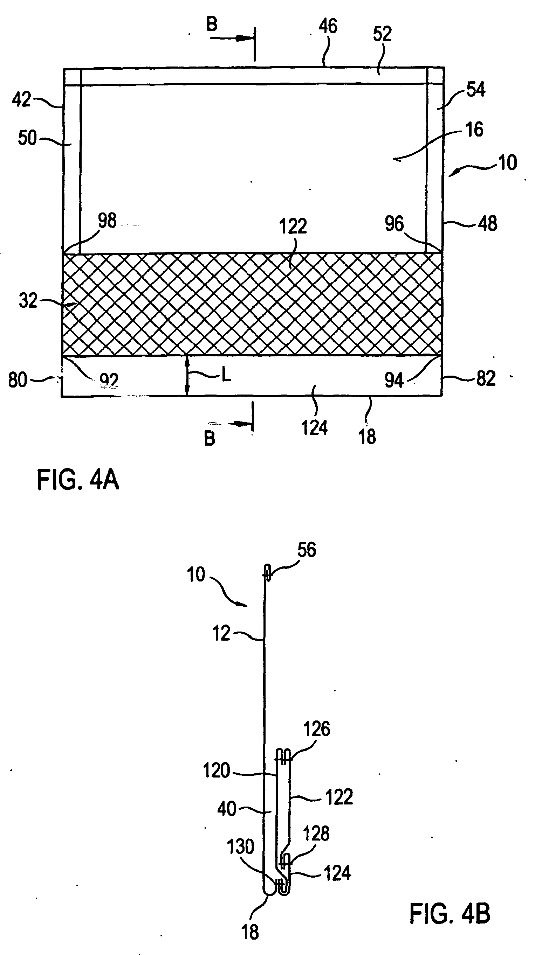

Turning now to Figs. 4A and 4B there can be seen a surgical drape with a structure similar to but differing from that of Figs. 3A and 3B. Here, the panel 12 again consists of the same material as described in connection with Figs. 1A and 1B, for example, ROCOT article no. 750.44.0.0. The panel 32 here comprises three different strips of material, namely a first strip 120 of a tightly woven medical fabric, such as ROTECNO 2000 which is liquid repellent and impervious to liquids, a second layer 122 of an absorbent medical fabric, such as ROTECNO sorbent, article no. 716.36.0.0 as described above and a third strip 124 of ROTECNO 2000 adjacent the edge 18 and disposed next to the wound to prevent liquids being transferred from the absorbent material 122 to the wound by wicking. The way in which the three panels 120, 122 and 124 are joined to each other and to the panel 12 can be seen from the schematically illustrated stitching in Fig. 4B. It can be seen that all the edges which are joined at the stitching 126, 128 and 130 are cut edges and these are conveniently each provided with a hemming stitch to prevent fraying.

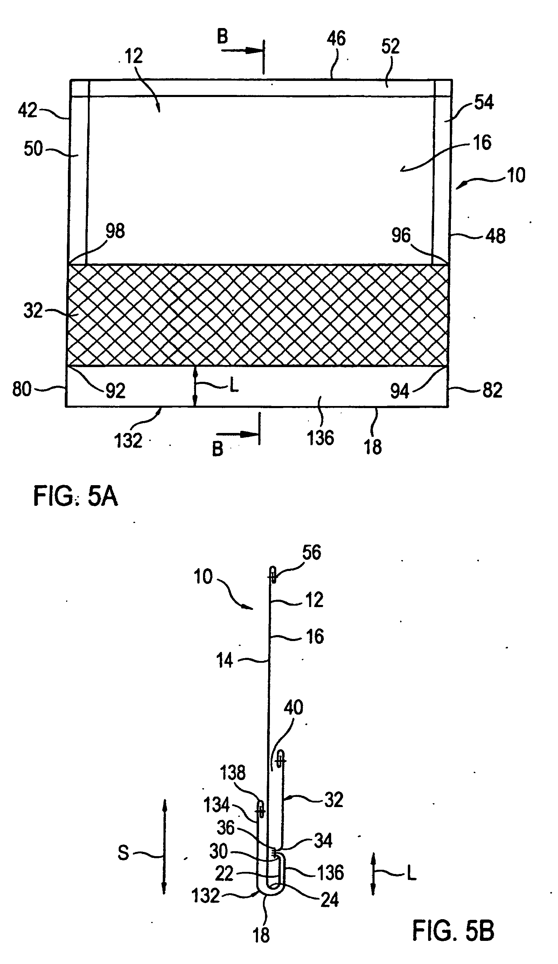

Turning now to Figs. 5A and 5B there can be seen an alternative design of a surgical drape in accordance with the present invention which is similar to that of Fig. 1, but differs from it in as much as an additional strip of material 132 is used to form a taping strip 134 and the folded back portion 136. The panel material 12 and the fabric 32 are arranged precisely as in the embodiment of Figs. 1A to 1E and made of the same materials as described with respect to that embodiment. The strip 132 is connected to the panel 12 and the fabric 32 at the seam shown by the stitching 36 and is sandwiched between the edges 30 and 34 of the panel 12 and the fabric 32 at this point. The fabric strip 132, which is otherwise provided with a folded hem 138 which is stitched right through, is folded around the fold 24 of the panel 12, so that the taping strip 134 is defined at the side of the folded back strip 32 adjacent the patient and facing in the same direction as the side 14 of the panel 12. Because the taping strip 134 is not sewn to the panel 12 in the region of the taping strip, it can be made of a different material from the panel 12 without the danger of differential shrinkage leading to creases and capillary passages. It can, however, also be made of the same material as the panel material 12. In any event, it should be impermeable to liquids. The length L of the folded back portion 136 of the strip 132 again preferably lies in the range from 2 to 5 cm, the length S of the taping strip 134 should preferably be at least 5 cm corresponding to the width of the conventionally used adhesive tape. Because there are two layers of liquid repellent material in the region of the folded edge 18, there is even less danger of bacteria transfer in this region.

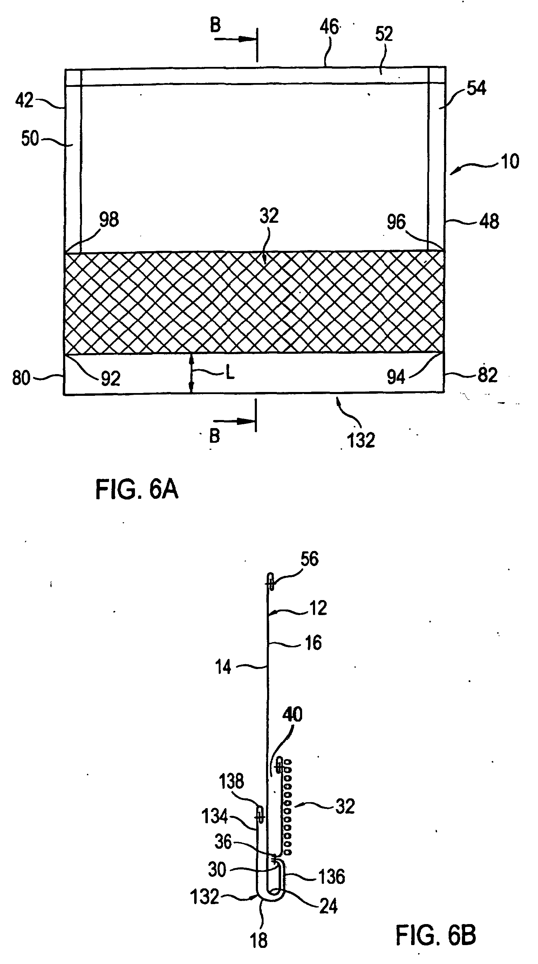

Turning now to Figs. 6A and 6B there can be seen a design of a surgical drape in accordance with the present invention which is closely similar to that of Figs. 5A and 5B, but in which the fabric 32 is formed by a laminate with an absorbent material on the outside. More specifically, the laminate here can be contrived in the same manner as the laminate of Fig. 2B, that is to say with respect to the choice of the materials for the laminate, it basically comprises a laminate with an absorbent surface and a liquid impermeable layer. The laminate of panel 32 is, however, in this case joined to the panel material 12 and to the taping strip 132 in the manner disclosed in connection with Fig. 5B.

As in the embodiment of Fig. 5B, the strip 132 can again be made of a different material from the material of the panel 12 and it can (as is also the case in the embodiment of Figs. 5A and 5B) also comprise a laminate of a repellent material with an outside surface or surface layer which is specifically designed to allow the tape to release during washing and also to be used with so-called soluble tape or with sprayed-on adhesive rather than tape. This feature aids in making use of the surgical drape more economical, because costly operations involving removing pieces of tape from the drapes can be avoided.

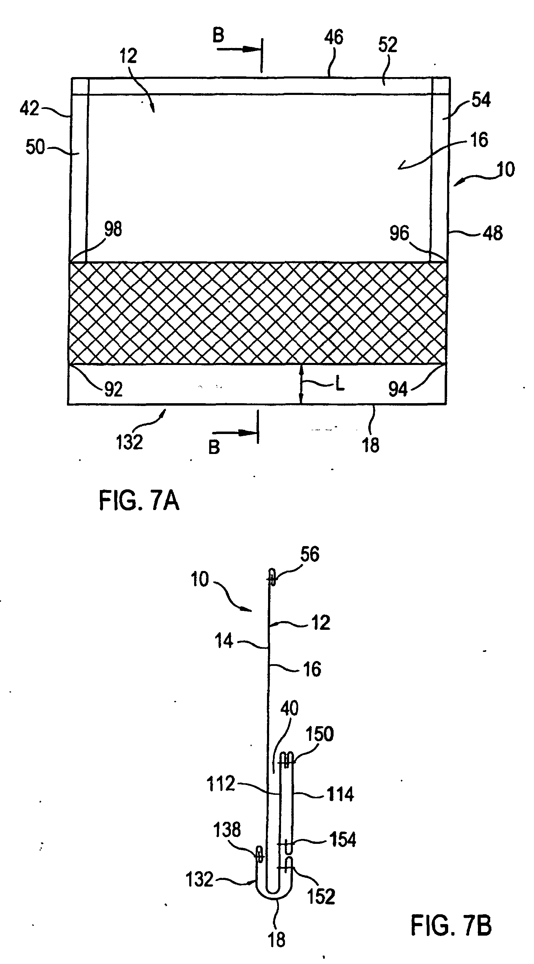

Finally, the embodiment of Figs. 7A and 7B shows a combination of an embodiment similar to Figs. 5A, 5B or 6A and 6B, with panel 32 contrived in accordance with Figs. 3A and 3B. Thus, the materials used for the panel 12, the layers 112 and 114 are as described for the embodiment of Figs. 3A and 3B and the material used for the taping strip 132 is one of the materials described in connection with the embodiments of Figs. 5A, 5B and 6A and 6B.

The way the stitching is contrived between the two layers of the panel material 12 and the strip 132 can be seen from the reference numerals 150, 152, 154.

## Claims

1. Surgical drape (10, 10') formed from at least one panel material (12) having a first side (14) disposed in use adjacent a patient and a second side (16) facing away from the patient, a taping strip (20; 134) at said first side (14) of said panel (12) and extending parallel to and adjacent to an edge (18) of said drape disposed in use adjacent to a site of a surgical wound (102), said taping strip being adhesively connectable in use to the patient (104), for example by the use of a two-sided adhesive tape (26), wherein the taping strip (20) comprises a single kind of medical fabric (12; 132), preferably a single layer of a medical fabric (12; 132), at least over a major portion of its width, having a weave or structure sufficiently dense to prevent the passage of bacteria through it, wherein the material (12; 132) forming the taping strip (20) has a portion (22; 136) folded back on itself at a fold (24) towards the second side (16) of the panel (12), and wherein any stitched seam (36) in the folded back portion (22; 136) does not pass through the panel material (12) directly adjacent to the patient **characterised in that** the material of the taping strip (20; 134) at the folded back portion (22;136) lies either directly opposite the material of the taping strip at said second side (16) or **in that** a double thickness of said panel material resulting from said fold (24) at said edge (18) of said drape lies between said taping strip (134) and said folded back portion (136) thereof.

2. Surgical drape (10, 10') in accordance with claim 1,
**characterised in that**
said taping strip (20) is defined by a zone of said panel (12) and said folded back portion (22) is a folded back portion of said panel (12), said fold (24) defining said edge (18).

3. Surgical drape (10, 10') in accordance with claim 2,
**characterised in that**
said folded back portion (22) of said panel is sewed at an edge zone (30) of said panel spaced from said edge (18) formed by the fold (24) of said panel to a further fabric or fabric structure (32) overlying a layer of said panel (12) adjacent to said patient (104).

4. Surgical drape (10, 10') in accordance with claim 3, wherein said further medical fabric or fabric structure (32) forms a pocket (40) with said layer of panel material (12) adjacent the patient (104).

5. Surgical drape (10, 10') in accordance with one of the claims 3 or 4,
wherein said further medical fabric (32) is an absorbent medical fabric (32; 114).

6. Surgical drape (10, 10') in accordance with claim 5, wherein said absorbent medical fabric (32) lies directly opposite to said second side (16) of said panel (12).

7. Surgical drape (10, 10') in accordance with claim 5, wherein said absorbent medical fabric (114) lies over a part (112) of said folded back portion of said panel (12).

8. Surgical drape (10, 10') in accordance with claim 3 or claim 4,
wherein said fabric structure has at least first and second layers (112; 114), a first liquid-repellent fabric layer (112) overlying the panel material (12) adjacent said patient (104) and a second layer (114) of an absorbent medical fabric.

9. Surgical drape (10, 10') in accordance with any one of the preceding claims,
wherein said fabric structure (32) is a laminate, for example a silicone laminate, having an absorbent surface.

10. Surgical drape (10, 10') in accordance with claim 9, wherein said panel (32) comprises a panel (120) of a tightly woven medical fabric impervious to bacteria, for example ROTECNO 2000, overlying said panel (12) adjacent to said patient (104), a panel (122) of absorbent material overlying said panel (120) of tightly woven medical fabric and a strip (100, 124) of tightly woven medical fabric adjacent said edge of said taping strip (20) and connected by a stitched seam (36, 128) to said absorbent fabric at a distance from said edge, said distance preferably being greater than 2 cm, but less than 6 cm.

11. Surgical drape (10, 10') in accordance with claim 1,
**characterised in that**
said taping strip (134) is formed by strip (132) of material folded around an edge fold (24) of said panel (12) and joined to a folded back portion (22) of said panel (12) at a position spaced by a distance (L) from said edge fold (24), said distance preferably being greater than 2cm but less than 6cm.

12. Surgical drape (10, 10') in accordance with claim 11,
**characterised in that**
said folded back portion (22) of said panel (12) is sewed at an edge zone (30) of said panel (12) spaced from an edge of said taping strip (134) formed by the fold of the strip of material (132) to a further fabric or fabric structure (32) overlying a layer of said panel (12) adjacent to said patient (104).

13. Surgical drape (10, 10') in accordance with claim 12, wherein said further medical fabric or fabric structure (32) forms a pocket (40) with said layer of panel material (12) adjacent the patient.

14. Surgical drape (10, 10') in accordance with one of the claims 12 or 13, wherein said further medical fabric (32) is an absorbent medical fabric.

15. Surgical drape (10, 10') in accordance with claim 14, wherein said absorbent medical fabric (32) lies directly opposite to said second side (16) of said panel (12).

16. Surgical drape (10, 10') in accordance with claim 12, wherein said absorbent medical fabric (114) lies over a part (112) of said folded back portion (22) of said panel (12).

17. Surgical drape (10, 10') in accordance with claim 12, wherein said fabric structure (32) has at least first and second layers, a first liquid-repellent fabric layer overlying the panel material adjacent said patient and a second layer of an absorbent medical fabric.

18. Surgical drape in accordance with any claim 12,
wherein said fabric structure (32) is a laminate, for example a silicone laminate, having an absorbent surface.

## Revendications

1. Champ opératoire (10, 10') formé à partir d'au moins un matériau de panneau (12) comportant une première face (14) disposée, en service, adjacente à un patient et une seconde face (16) tournée du côté opposé au patient, une bande collante (20 ; 134) au niveau de ladite première face (14) dudit panneau (12) et s'étendant parallèlement à, et adjacente à, un bord (18) dudit champ disposé, en service, adjacent à un site d'une plaie chirurgicale (102), ladite bande collante pouvant être reliée de façon adhésive, en service, au patient (104), par exemple en utilisant un ruban adhésif double face (26), dans lequel la bande collante (20) comprend un seul type de tissu médical (12 ; 132), de préférence une seule couche de tissu médical (12 ; 132), au moins sur une majeure partie de sa largeur, comportant une armure ou une structure suffisamment dense pour empêcher le passage des bactéries à travers elle, dans lequel le matériau (12 ; 132) formant la bande collante (20) comporte une partie (22 ; 136) repliée sur elle-même au niveau d'un pli (24) vers la seconde face (16) du panneau (12), et dans lequel toute couture piquée (36) dans la partie repliée (22 ; 136) ne traverse pas le matériau de panneau (12) directement adjacent au patient, **caractérisé en ce que** le matériau de la bande collante (20 ; 134) au niveau de la partie repliée (22 ; 136) se situe directement à l'opposé du matériau de la bande collante au niveau de la seconde face (16), ou **en ce qu'**une double épaisseur dudit matériau de panneau résultant dudit pli (24) au niveau dudit bord (18) dudit champ se situe entre ladite bande collante (134) et sa partie repliée (136).

2. Champ opératoire (10, 10') selon la revendication 1, **caractérisé en ce que**
ladite bande collante (20) est définie par une zone dudit panneau (12) et ladite partie repliée (22) est une partie repliée dudit panneau (12), ledit pli (24) définissant ledit bord (18).

3. Champ opératoire (10, 10') selon la revendication 2, **caractérisé en ce que**
ladite partie repliée (22) dudit panneau est cousue, au niveau d'une zone de bord (30) dudit panneau espacée dudit bord (18) formé par le pli (24) dudit panneau, à une structure de tissu supplémentaire ou à un tissu supplémentaire (32) recouvrant une couche dudit panneau (12) adjacente audit patient (104).

4. Champ opératoire (10, 10') selon la revendication 3, dans lequel ladite structure de tissu médical supplémentaire ou ledit tissu médical supplémentaire (32) forme une poche (40) avec ladite couche de matériau de panneau (12) adjacente au patient (104).

5. Champ opératoire (10, 10') selon l'une quelconque des revendications 3 ou 4, dans lequel ledit tissu médical supplémentaire (32) est un tissu médical absorbant (32 ; 114).

6. Champ opératoire (10, 10') selon la revendication 5, dans lequel ledit tissu médical absorbant (32) se situe directement à l'opposé de ladite seconde face (16) dudit panneau (12).

7. Champ opératoire (10, 10') selon la revendication 5, dans lequel ledit tissu médical absorbant (114) se situe sur une partie (112) de ladite partie repliée dudit panneau (12).

8. Champ opératoire (10, 10') selon la revendication 3 ou la revendication 4, dans lequel ladite structure de tissu comporte au moins des première et seconde couches (112 ; 114), une première couche de tissu repoussant les liquides (112) recouvrant le matériau de panneau (12) adjacent audit patient (104) et une seconde couche (114) d'un tissu médical absorbant.

9. Champ opératoire (10, 10') selon l'une quelconque des revendications précédentes, dans lequel ladite structure de tissu (32) est un laminé, par exemple un laminé de silicone, comportant une surface absorbante.

10. Champ opératoire (10, 10') selon la revendication 9, dans lequel ledit panneau (32) comprend un panneau (120) d'un tissu médical tissé serré, imperméable aux bactéries, par exemple ROTECNO 2000, recouvrant ledit panneau (12) adjacent audit patient (104), un panneau (122) de matériau absorbant recouvrant ledit panneau (120) de tissu médical tissé serré et une bande (100, 124) de tissu médical tissé serré adjacente audit bord de ladite bande collante (20) et reliée par une couture piquée (36, 128) audit tissu absorbant à une distance dudit bord, ladite distance étant, de préférence, supérieure à 2 cm mais inférieure à 6 cm.

11. Champ opératoire (10, 10') selon la revendication 1, **caractérisé en ce que**
ladite bande collante (134) est formée par une bande (132) de matériau pliée autour d'un pli de bord (24) dudit panneau (12) et reliée à une partie repliée (22) dudit panneau (12) en une position espacée d'une distance (L) dudit pli de bord (24), ladite distance étant, de préférence, supérieure à 2 cm mais inférieure à 6 cm.

12. Champ opératoire (10, 10') selon la revendication 11, **caractérisé en ce que**
ladite partie repliée (22) dudit panneau (12) est cousue, au niveau d'une zone de bord (30) dudit panneau (12) espacée d'un bord de ladite bande collante (134) formée par le pli de la bande de matériau (132), à une structure de tissu supplémentaire ou à un tissu supplémentaire (32) recouvrant une couche dudit panneau (12) adjacente audit patient (104).

13. Champ opératoire (10, 10') selon la revendication 12, dans lequel ladite structure de tissu médical supplémentaire ou ledit tissu médical supplémentaire (32) forme une poche (40) avec ladite couche de matériau de panneau (12) adjacente au patient.

14. Champ opératoire (10, 10') selon l'une quelconque des revendications 12 ou 13, dans lequel ledit tissu médical supplémentaire (32) est un tissu médical absorbant.

15. Champ opératoire (10, 10') selon la revendication 14, dans lequel ledit tissu médical absorbant (32) se situe directement à l'opposé de ladite seconde face (16) dudit panneau (12).

16. Champ opératoire (10, 10') selon la revendication 12, dans lequel ledit tissu médical absorbant (114) se situe sur une partie (112) de ladite partie repliée (22) dudit panneau (12).

17. Champ opératoire (10, 10') selon la revendication 12, dans lequel ladite structure de tissu (32) comporte au moins des première et seconde couches, une première couche de tissu repoussant les liquides recouvrant le matériau de panneau adjacent audit patient et une seconde couche d'un tissu médical absorbant.

18. Champ opératoire selon la revendication 12, dans lequel ladite structure de tissu (32) est un laminé, par exemple un laminé de silicone, comportant une surface absorbante.

## Patentansprüche

1. Operationstuch (10, 10'), das aus zumindest einem Bahnmaterial (12), das eine erste Seite (14), die im Gebrauch benachbart eines Patienten angeordnet ist, und eine zweite Seite (16) besitzt, die von dem Patienten weg weist, und einem Anklebestreifen (20; 134) an der ersten Seite (14) der Bahn (12) geformt ist, der sich parallel zu und benachbart eines Randes (18) des Tuchs erstreckt, der im Gebrauch benachbart eines Ortes einer Operationswunde (102) angeordnet ist, wobei der Anklebestreifen im Gebrauch mit dem Patienten (104) klebend verbindbar ist, beispielsweise durch die Verwendung eines zweiseitigen Klebebandes (26), wobei der Anklebestreifen (20) einen einzelnen Typ medizinischen Gewebes (12; 132), bevorzugt eine einzelne Lage eines medizinischen Gewebes (12; 132), zumindest über einen Großteil seiner Breite mit einem Webmuster oder einer Struktur umfasst, die ausreichend dicht ist, um den Durchgang von Bakterien durch dieses zu verhindern, wobei das Material (12; 132), das den Anklebestreifen (20) bildet, einen Abschnitt (22; 136) aufweist, der auf sich selbst an einer Falte (24) in Richtung der zweiten Seite (16) der Bahn (12) zurückgefaltet ist, und wobei jede Naht (36) in dem zurückgefalteten Abschnitt (22; 136) nicht durch das Bahnmaterial (12) direkt benachbart des Patienten verläuft,
**dadurch gekennzeichnet,**
**dass** das Material des Anklebestreifens (20; 134) an dem zurückgefalteten Abschnitt (22; 136) entweder direkt entgegengesetzt des Materials des Anklebestreifens an der zweiten Seite (16) liegt, oder dass eine Doppeldicke des Bahnmaterials, die aus der Faltung (24) an dem Rand (18) des Tuchs resultiert, zwischen dem Anklebestreifen (134) und dem zurückgefalteten Abschnitt (136) desselben liegt.

2. Operationstuch (10, 10') nach Anspruch 1,
**dadurch gekennzeichnet,**
**dass** der Anklebestreifen (20) durch eine Zone der Bahn (12) definiert ist und der zurückgefaltete Abschnitt (22) ein zurückgefalteter Abschnitt der Bahn (12) ist, wobei die Faltung (24) den Rand (18) definiert.

3. Operationstuch (10, 10') nach Anspruch 2,
**dadurch gekennzeichnet,**
**dass** der zurückgefaltete Abschnitt (22) der Bahn an einer Randzone (30) der Bahn beabstandet von dem Rand (18), der durch die Faltung (24) der Bahn ausgebildet wird, mit einem weiteren Gewebe oder mit einer weiteren Gewebestruktur (32) vernäht ist, die über einer Lage der Bahn (12) benachbart des Patienten (104) liegt.

4. Operationstuch (10, 10') nach Anspruch 3,
wobei das weitere medizinische Gewebe oder die weitere medizinische Gewebestruktur (32) eine Tasche (40) mit der Lage des Bahnmaterials (12) benachbart des Patienten (104) bildet.

5. Operationstuch (10, 10') nach einem der Ansprüche 3 oder 4,
wobei das weitere medizinische Gewebe (32) ein absorbierendes medizinisches Gewebe (32; 114) ist.

6. Operationstuch (10, 10') nach Anspruch 5,
wobei das absorbierende medizinische Gewebe (32) direkt entgegengesetzt der zweiten Seite (16) der Bahn (12) liegt.

7. Operationstuch (10, 10') nach Anspruch 5,
wobei das absorbierende medizinische Gewebe (114) über einem Teil (112) des zurückgefalteten Abschnitts der Bahn (12) liegt.

8. Operationstuch (10, 10') nach einem der Ansprüche 3 oder 4,
wobei die Gewebestruktur zumindest erste und zweite Lagen (112; 114), eine erste flüssigkeitsabweisende Gewebelage (112), die über dem Bahnmaterial (12) benachbart des Patienten (104) liegt, und eine zweite Lage (114) eines absorbierenden medizinischen Gewebes besitzt.

9. Operationstuch (10, 10') nach einem der vorhergehenden Ansprüche,
wobei die Gewebestruktur (32) ein Verbundstoff ist, beispielsweise ein Silikonverbundstoff mit einer absorbierenden Oberfläche.

10. Operationstuch (10, 10') nach Anspruch 9,
wobei die Bahn (32) eine Bahn (120) aus einem eng gewobenen medizinischen Gewebe, das für Bakterien undurchdringlich ist, beispielsweise ROTECNO 2000, die über der Bahn (12) benachbart des Patienten (104) liegt, eine Bahn (122) aus absorbierendem Material, die über der Bahn (120) aus eng gewobenem medizinischem Gewebe liegt, und einen Streifen (100, 124) aus eng gewobenem medizinischem Gewebe benachbart des Randes des Anklebestreifens (20) umfasst, der durch eine Naht (36, 128) mit dem absorbierenden Gewebe in einer Distanz von dem Rand verbunden ist, wobei die Distanz bevorzugt größer als 2 cm, jedoch kleiner als 6 cm ist.

11. Operationstuch (10, 10') nach Anspruch 1,
**dadurch gekennzeichnet,**
**dass** der Anklebestreifen (134) durch einen Streifen (132) aus einem Material geformt ist, der um eine Randfaltung (24) der Bahn (12) gefaltet ist und mit einem rückgefalteten Abschnitt (22), der Bahn (12) an einer Position verbunden ist, die um eine Distanz (L) von der Randfaltung (24) beabstandet ist, wobei die Distanz bevorzugt größer als 2 cm, jedoch kleiner als 6 cm ist.

12. Operationstuch (10, 10') nach Anspruch 1,
**dadurch gekennzeichnet,**
**dass** der zurückgefaltete Abschnitt (22) der Bahn (12) an einer Randzone (30) der Bahn (12), die von einem Rand des Anklebestreifens (134) beabstandet ist, der durch die Faltung des Streifens aus Material (132) gebildet ist, mit einem weiteren Gewebe oder einer weiteren Gewebestruktur (32) vernäht ist, die über einer Lage der Bahn (12) benachbart des Patienten (104) liegt.

13. Operationstuch (10, 10') nach Anspruch 12,
wobei das weitere medizinische Gewebe oder die weitere medizinische Gewebestruktur (32) eine Tasche (40) mit der Lage aus Bahnmaterial (12) benachbart des Patienten bildet.

14. Operationstuch (10, 10') nach einem der Ansprüche 12 oder 13,
wobei das weitere medizinische Gewebe (32) ein absorbierendes medizinisches Gewebe ist.

15. Operationstuch (10, 10') nach Anspruch 14,
wobei das absorbierende medizinische Gewebe (32) direkt entgegengesetzt der zweiten Seite (16) der Bahn (12) liegt.

16. Operationstuch (10, 10') nach Anspruch 12,
wobei das absorbierende medizinische Gewebe (114) über einem Teil (112) des zurückgefalteten Abschnitts (22) der Bahn (12) liegt.

17. Operationstuch (10, 10') nach Anspruch 12,
wobei die Gewebestruktur (32) zumindest eine erste und zweite Lage, eine erste flüssigkeitsabweisende Gewebelage, die über dem Bahnmaterial benachbart des Patienten liegt, und eine zweite Lage eines absorbierenden medizinischen Gewebes umfasst.

18. Operationstuch (10, 10') nach Anspruch 12,
wobei die Gewebestruktur (32) ein Verbundstoff ist, beispielsweise ein Silikonverbundstoff mit einer absorbierenden Oberfläche.
